# EUROPEAN PATENT APPLICATION

(11) **EP 4 806 449 A1**
(43) Date of publication of application: **16.09.2026**
(21) Application number: 24911652.6
(22) Date of filing: 24.12.2024
(51) Int. Cl.: A61K 36/87, A61P 31/12

(54) **ANTIVIRAL FOOD PRODUCT**

(30) Priority: 26.12.2023 ES 202331086
(71) Applicant: Creaciones Aromáticas Industriales, S.A., 08192 Sant Quirze del Vallès, Barcelona (ES)
(72) Inventor: PALOMES ROYO, Ángel, 08192 Sant Quirze del Vallès (Barcelona) (ES); COSTA BARRES, Arturo, 08150 Parets del Vallès (Barcelona) (ES); MARTÍNEZ CHAMORRO, Vanesa, 08017 Barcelona (ES)
(74) Representative: Pons IP
(86) International application number: PCT/ES2024/070807
(87) International publication number: WO 2025/141238

(57) **Abstract**

The present invention relates, in a first aspect, to an oral antiviral product, characterised in that it comprises black grape extract. Preferably, this oral product has the grape extract encapsulated in a capsule selected from the group consisting of a capsule with maltodextrin, a capsule with gelatin and a combination of the two. The invention also relates to the use of an oral product comprising black grape extract as an antiviral agent.

## Description

### TECHNICAL SECTOR

The present invention falls within the sector of food products, and particularly, it relates to an oral product capable of reducing and/or eliminating the viral load in the mouth. Therefore, this invention also falls within the field of microbiology, and specifically, in the field of viral infections and antiviral compositions.

### BACKGROUND OF THE INVENTION

Viral transmission is one of the most important factors considered in the study of infectious diseases. Most viruses are transmitted orally. When a person coughs, speaks or sneezes, they exhale a series of saliva droplets or particles into the environment, which have a high infectious power.

Various studies show that ambient temperature and relative humidity are factors that strongly influence the evaporation process. In turn, the evaporation time depends on the particle size, thus, the smaller the diameter, less evaporation time is required. Furthermore, it is known that these very small particles are capable of remaining suspended in the air for a time comprised from 60-70 seconds, which allows them to be able to travel a certain distance. (Computational characterization of the behavior of a saliva droplet in a social environment. Ainara Ugarte-Anero, Unai Fernandez-Gamiz, Koldo Portal-Porras, Ekaitz Zulueta & Oskar Urbina-Garcia. Scientific Reports volume 12, Article number: 6405 (2022)).

On this basis, it seems reasonable to think that those microparticles that an individual has in their mouth could travel and are capable of infecting other individuals.

It is well known that, due to their nature, viruses are transmitted through these saliva droplets or aerosols, but also by means of contact with oral and nasal mucous membranes, infecting a subject by entering the nasal or respiratory tract.

Viruses are known to be the main factors responsible for the most common diseases affecting the human population on an annual basis. There are some 219 species of viruses capable of infecting human beings, and 3-4 new ones are discovered every year, the most frequent being *rhinovirus* and *parainfluenza* virus, *influenzavirus A* or *influenzavirus B* (both belonging to the *Orthomyxoviridae* family), the *HIV* virus, the virus of the *paramyxovirus* family, *hepatitis A and B* virus, *poliovirus*, or *SARS-CoV-2* virus. These microorganisms are responsible for common illnesses and infections such as colds, flu or warts. Nevertheless, they are also responsible for very serious diseases such as HIV, Ebola, AIDS and COVID-19, which is responsible for the pandemic that started in 2020.

To that end, in parallel to the development of effective treatments for these viral diseases, different methodologies that intervene in the transmission process, thus preventing a subject from becoming ill, are also being developed.

It has been found that reducing the viral load in the mouth and oesophagus is a possible option. In fact, there are different products known in the state of the art the object of which is to reduce the viral load in the oral cavity.

Among them is a spray against Covid infection that is applied in the nasal cavities and has been developed by a research group at the Spanish National Research Council (CSIC-Spain). It should be noted that the active ingredient of this spray is hypromellose (hydroxypropyl methylcellulose, and its acronym HPMC) which, together with citric acid, sodium citrate, and benzalkonium chloride acts against SARS-Cov. An anti-virus chewing gum developed by the German laboratory Clevergum GmbH is also known. Said product contains essential oils such as cinnamon bark, mint and lemon; ginseng; quercetin; zinc, and other ingredients such as ginger extract, vitamin D3, sucralose, spermidine (wheat germ extract), xylitol, medicinal gum base, natural colourant and natural flavour.

These products comprise a combination of acids, the most important of which is acetic acid, which acts on the outer lipid capsid of the virus and other components that interfere with coronavirus spicules, which inactivates the infectious activity of the virus.

Also known in the state of the art is international patent application WO2022241010A1 describing a composition and method for reducing viral load in the oral cavity, particularly Coronavirus such as SARS-CoV-2 and Influenza viral loads, comprising a trapping molecule having binding affinity for a protein, glucan or other molecule on the surface of a virus or microorganism.

Nevertheless, consumers of these products report that the taste is very bitter, so ultimately, these products are rejected by final consumers.

That is why there is a need to discover new edible products aimed at reducing, and even effectively eliminate the viral load in the oral and oesophageal cavity, in order to prevent the transmission of viral pathogens, and that furthermore has a pleasant taste for the end consumer.

### DESCRIPTION OF THE INVENTION

### BRIEF DESCRIPTION OF THE INVENTION

In a first embodiment, the invention relates to an oral antiviral product, characterised in that it comprises black grape extract, wherein the grape extract is in free form and/or encapsulated in a capsule of the type selected from the group consisting of a capsule with maltodextrin, a capsule with gelatin and a combination of the two, and wherein said oral product is presented in a soluble or chewable form inside the oral cavity.

In a second embodiment, the invention relates to an oral product for use as an antiviral agent.

In further embodiments, the composition of the invention for use in the prevention and/or treatment of coronavirus infection is described.

In another embodiment, the composition of the invention for use in reducing viral load in the mouth is described.

In the present document, when grape extract is indicated, it refers to black grape extract.

Furthermore, in the present document, all percentages described refer to percentages by weight with respect to the total weight.

### BRIEF DESCRIPTION OF THE DRAWINGS

To complement the description that is being made and for the purpose of helping to better understand the features of the invention, a set of figures is attached as an integral part of said description wherein the following has been depicted with an illustrative and non-limiting character:

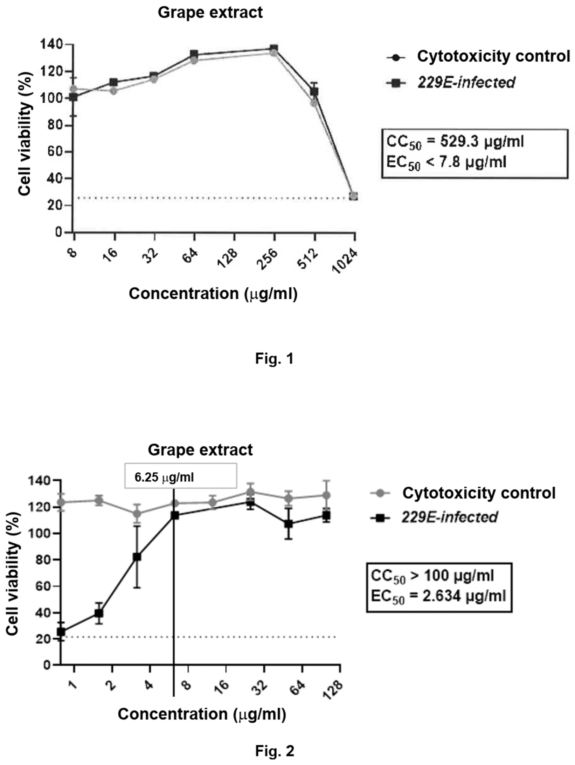

Figure 1. Example 1 - Experiment 1. Cell viability, determined by means of MTT, of MRC-5 cells treated with the composition under study comprising different concentrations (7.81-1000 µg/ml) of the grape extract with the presence (black line and dots) or absence (grey line and dots) of an inoculum of 229E. The discontinuous line marks the mean viability of infected MRC-5 cells without any treatment. CC₅₀: half maximal cytotoxic concentration; EC₅₀: half maximal effective concentration.
Figure 2. Example 1 - Experiment 2. Cell viability, determined by means of MTT, of MRC-5 cells treated with the composition under study comprising different concentrations (0,781-100 µg/ml) of the grape extract with the presence (black line and dots) or absence (grey line and dots) of 229E inoculum. The discontinuous line marks the mean viability of infected MRC-5 cells without any treatment. CC₅₀: half maximal cytotoxic concentration; EC₅₀: half maximal effective concentration.

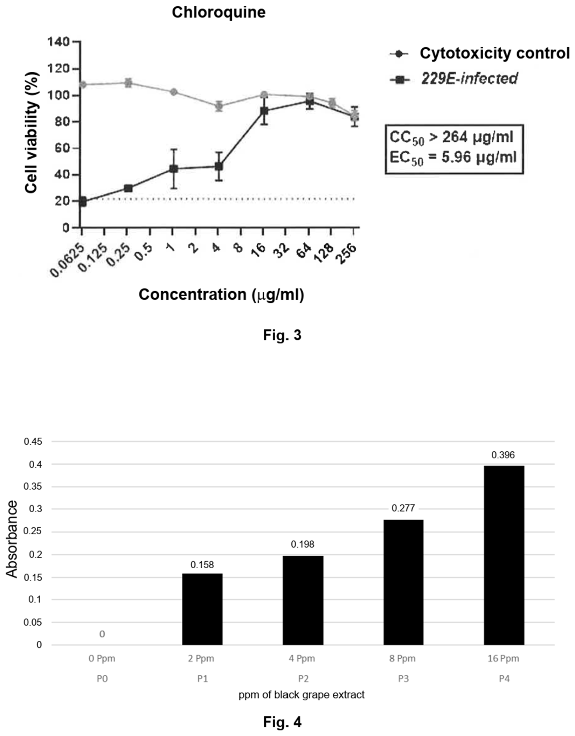

Figure 3. Example 1 - Cell viability, determined by means of MTT, of MRC-5 cells treated with different concentrations of chloroquine with the presence (black line and dots) or absence (grey line and dots) of 229E inoculum. The discontinuous line marks the mean viability of infected MRC-5 cells without any treatment. CC₅₀: half maximal cytotoxic concentration; EC₅₀: half maximal effective concentration.
Figure 4. The linear regression between absorbance and ppm of black grape extract is shown.

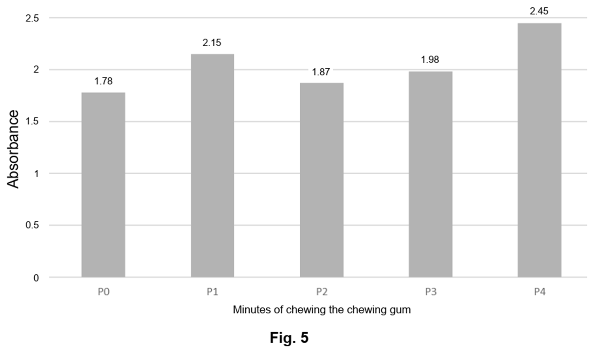

Figure 5. Result where the absorbance, obtained by the spectrophotometric method, of saliva extracts obtained from chewing gum at different chewing times, can be observed. P0: 2 minutes; P1: 5 minutes; P2: 10 minutes; P3: 18 minutes; P4: 30 minutes.

### DETAILED DESCRIPTION OF THE INVENTION

In a first aspect, the present invention provides an oral antiviral product comprising black grape extract, and wherein said oral product is presented in a form selected from the group consisting of mouth-soluble or chewable form.

Therefore, an object of the present invention is an oral antiviral product, characterised in that it comprises black grape extract, wherein the grape extract is in free form and/or encapsulated in a capsule of the type selected from the group consisting of a capsule with maltodextrin, a capsule with gelatin and a combination of the two.

In a preferred embodiment, the oral product is in a soluble or chewable form.

After numerous studies, the inventors have developed an oral product that is held in the oral cavity for chewing or sucking in the oral cavity, releasing an active ingredient in a controlled manner in significantly reduced amounts, which active ingredient is black grape extract and is capable of inhibiting and even eliminating a viral-type microorganism. In preferred embodiments, this viral organism is the coronavirus.

In the context of the present invention, a soluble form is a product wherein its components dissolve in a liquid medium to be incorporated into said liquid medium.

In the context of the present invention, a chewable form is a product that can be chewed, or, in other words, can be crushed with the teeth, palate and tongue to release its components into the oral cavity.

In particular embodiments of the present invention, the oral antiviral product comprises black grape extract, wherein the grape extract is in free form and/or encapsulated in a capsule of the type selected from the group consisting of a capsule with maltodextrin, a capsule with gelatin and a combination of the two.

In particular embodiments of the present invention, the black grape extract comprised in the oral product object of the invention is characterised in that in the analysis of total polyphenols, it shows 100% catechin equivalents in a 280nm OD analysis. In a particular embodiment of the invention, the grape extract is characterised in that in the analysis of total polyphenols, it exhibits 70% gallic acid equivalents in a Folin-Ciacalteu analysis. In another particular embodiment of the invention, the grape extract is characterised in that in the analysis of total phenols, it exhibits 2% anthocyanin (cyanidin-3-O-glucoside) equivalents in an HPLC-DAD analysis.

In the context of the present invention, catechin is a polyphenolic antioxidant. The term catechin is commonly used to refer to the flavonoid family and the flavan-3-ol subgroup.

Furthermore, in the context of the present invention, gallic acid is a phenolic compound found in a various natural sources such as plants, fruit and vegetables.

Lastly, in the context of the present invention, anthocyanin is a pigment found in many red berries, for example in grapes, among others.

The black grape extract comprised in the oral product comprises, in turn, a combination of anthocyanin with one or more polyphenols. In a preferred embodiment, the at least one polyphenol comprised in the grape extract is selected from the group consisting of phenolic acid, stilbene, lignan, phenolic alcohol, flavonoid or a combination thereof.

In more preferred embodiments of the present invention, the phenolic acid comprised in the black grape extract is an acid derived from hydroxybenzoic acid or hydroxycinnamic acid.

In a preferred embodiment, the oral product comprises free black grape extract in an amount of 0.0006 - 9% by weight with respect to the total weight of the oral product. In a more preferred embodiment, 0.06 - 4.5% by weight with respect to the total weight of the oral product.

In other particular embodiments of the present invention, the oral product comprises grape extract in an amount of 0.06%, 0.07%, 0.08%, 0.09%, 0.1%, 0.2%, 0.3%, 0.4%, 0.5%, 0.6%, 0.7%, 0.8%, 0.9% or 1%. In preferred embodiments of the present invention, the oral product comprises grape extract in an amount of 1%, 1.1%, 1.2%, 1.3%, 1.4%, 1.5%, 1.6%, 1.7%, 1.8%, 1.9% or 2% by weight with respect to the total weight.

In other particular embodiments of the present invention, the oral product comprises grape extract in an amount of 2%, 2.5%, 3%, 3.5%, 4%, 4.5%, 5%, 5.5%, 6%, 6.5%, 7%, 7.5%, 8%, 8.5% or 9% by weight with respect to the total weight.

In preferred embodiments of the present invention, the oral product object of the invention comprises the grape extract encapsulated in a capsule of the type selected from the group consisting of a capsule with maltodextrin, a capsule with gelatin and a combination of the two.

In another preferred embodiment, the oral product comprises grape extract which is encapsulated in a capsule with maltodextrin. In the context of the present invention, grape extract encapsulated in a capsule with maltodextrin is referred to as type A capsule.

In a more preferred embodiment of the present invention, the oral product comprises a grape extract encapsulated in a type A capsule and comprises the following components and amounts:

**Table 1. Components and amounts of type A capsules.**

| | preferred amount (% by weight) | more preferred (% by weight) | the most preferred (% by weight) |
|---|---|---|---|
| Black grape extract | 1% - 80% | 20% - 60% | 39% |
| Acacia gum | 9% - 44% | 18% - 35% | 27% |
| Modified starch | 3% - 13% | 5% - 11% | 7% |
| Maltodextrin | 9% - 42% | 17% - 34% | 27% |

In an even more preferred embodiment, the oral product comprises black grape extract encapsulated in a type A capsule having the following composition:
- 38.46% black grape extract
- 26.92% acacia gum
- 7.7% modified starch
- 26.92% maltodextrin

The inventors have found that the presence of maltodextrin influences the solubility of the grape extract, and that is why it directly influences the release of grape extract within the oral cavity when the oral product is tasted or chewed.

The present invention uses different types of maltodextrin comprised in the type A capsule depending on the level of hydrolysis of the dextrose chains provided by starch, so it may be derived from corn, potato, sweet potato, tapioca, etc. According to the level of hydrolysis, maltodextrin can have a dextrose equivalent (DE) of 2, 6, 12, 19. The higher the dextrose equivalent, the greater the hydrolysis of maltodextrin. In a more preferred embodiment of the present invention, the dextrose equivalent is DE = 19.

The presence of maltodextrin influences the release because maltodextrin, due to its chemical nature, dissolves readily in water.

In another preferred embodiment, the oral product comprises black grape extract encapsulated in a gelatin capsule. Grape extract encapsulated in a gelatin capsule, which, in the context of the present invention, is referred to as type B capsule, is less soluble than the capsule with maltodextrin. In a more preferred embodiment of the present invention, the oral product comprises a grape extract encapsulated in a type B capsule and comprises the following components and amounts:

**Table 2. Components and amounts of type B capsules.**

| | Preferred embodiment (%) | More preferred embodiment (%) | Even more preferred embodiment (%) |
|---|---|---|---|
| Black grape extract | 1% - 80% | 20% - 60% | 44% |
| Acacia gum | 12% - 58% | 23% - 47% | 33% |
| Modified starch | 4% - 21% | 9% - 17% | 12% |
| Gelatin | 4% - 20% | 8% - 16% | 11% |

In an even more preferred embodiment, the oral product comprises black grape extract encapsulated in a type B capsule having the following composition:
- 44.29% black grape extract
- 33.22% acacia gum
- 11.42% modified starch
- 11.07% gelatin

Gelatin can have different gelling intensities. In the context of the present invention, gelling intensity is measured by Bloom grade. The higher the Bloom grade, the higher the gel strength, therefore, the lower the hydrolysis. Bloom grades usually range from about 80° Bloom to about 380° Bloom, preferably 250° Bloom.

To that end, the oral product described here has the surprising effect of releasing a minimum effective amount of black grape extract in a constant and controlled manner, allowing the viral load in the oral cavity to be eliminated, without the need to reach high doses which can be toxic for the individual ingesting the oral product object of the invention.

In particular embodiments of the present invention, the grape extract in a type A encapsulation is released at a higher rate compared to the grape extract in a type B encapsulation. In particular embodiments of the invention, the inventors have found that the encapsulated grape extract is released more slowly than the grape extract in free form when the oral product object of the invention is ingested, with a constant and progressive release being even more preferred when the oral product is a chewing gum and is chewed.

Thanks to numerous studies, the inventors have found that in the preferred embodiment in which the oral product is a chewing gum comprising black grape extract encapsulated in type A and type B capsules, after 30 minutes of chewing, the chewing gum continues to release enough black grape extract to eliminate viral microorganisms. Furthermore, after these 30 minutes have elapsed, and up to a maximum of 60 minutes, black grape extract could be detected in the test area, which demonstrates that the chewing gum is still effective for a time exceeding the mean time during which users typically chew chewing gum.

In a particular embodiment of the invention, the oral product is a confectionery product selected from the group consisting of a chewing gum, a lozenge, a gummy, a gel and a candy.

In the context of the present invention, chewing gum is understood to mean a flavoured chewable product that is not swallowed, with a rubber-like texture, which releases a substance during chewing.

In the context of the present invention, a lozenge is understood to mean a very small portion of hard paste composed mainly of sugar and some substance pleasant to the taste.

In the context of the present invention, gummy is understood to mean a portion of a given size of soft paste composed mainly of sugar, glucose syrup, gelling agents, wetting agents, flavourings, colourants, which make this product pleasant to the taste.

In the context of the present invention, gel is understood to mean a portion of paste which takes on a colloidal dispersion of matter when it sets.

In the context of the present invention, candy is understood to mean a small portion of medicinal material in a generally round shape, and coated with a layer of a palatable substance which, in the present invention, comprises an effective amount of the antiviral composition. In preferred embodiments of the present invention, the candy is a lollipop, which, in the context of the present invention, is a candy arranged at the end of a stick. This type of product has the advantage that the stick prevents the candy from being swallowed, preventing choking.

In a preferred embodiment of the present invention, the oral product is a chewing gum, and in preferred embodiments, it is a dragée-type chewing gum. In the context of the present invention, a dragée-type chewing gum is a chewing gum in lozenge form, comprising a central core of chewing gum and a coating over its entire surface.

In particular embodiments of the present invention, the oral product is a dragée-type chewing gum and has the following composition:
Coating: 30% by weight with respect to the total weight of the chewing gum and in turn comprising:
- 86.84% polyols;
- 9.29% solution of 45% acacia gum in water;
- 0.13% artificial sweeteners;
- 1.59% flavourings;
- 1.59% acids;
- 0.56% glazing agents.
Chewing gum centre: 70% by weight with respect to the total weight of the chewing gum and in turn comprising:
- 52.48% polyols
- 35.00% gum base
- 5.65% flavourings
- 3.12% acids
- 0.60% high-intensity sweeteners
- 0.15% emulsifiers
- 1.00% free black grape extract
- 1.00% P.P. ENOCAPSUL A: Encapsulated, fast-dissolving black grape extract
- 1.00% P.P. ENOCAPSUL B: Encapsulated, slow-dissolving black grape extract

In another preferred embodiment of the present invention, the oral product is a chewing gum object of the invention comprising:
- 62.33% polyols, preferably, sorbitol, maltitol and mannitol syrup;
- 27.00% gum base;
- 3.00% wetting agent, preferably: glycerine;
- 2.45% flavourings;
- 2.60% acids, preferably: citric acid and malic acid;
- 0.60% free black grape extract;
- 0.60% P.P. ENCAPSUL A: encapsulated, rapid-release black grape extract;
- 0.60% P.P. ENCAPSUL B: encapsulated, slow-release black grape extract;
- 0.72% sweetener, preferably, aspartame, sucralose and acesulfame K;
- 0.10% emulsifier, preferably: sunflower lecithin.

In a preferred embodiment of the present invention, the oral product is a hard candy comprising the following composition:

**Table 3. Table describing the amounts in which each of the components is comprised for a preferred embodiment, a more preferred embodiment and an even more preferred embodiment wherein the oral product is a standard hard candy.**

| **STANDARD HARD CANDY WITH BLACK GRAPE EXTRACT** | | | |
|---|---|---|---|
| Product ingredients: With sugar/Sugar-free | A preferred embodiment (% by weight) | A more preferred embodiment (% by weight) | An even more preferred embodiment (% by weight) |
| Sugar/Polyols | ---- - ---- | ---- - ---- | 48.23 |
| Glucose syrup/Maltitol syrup | ---- - ---- | ---- - ---- | 48.23 |
| Acids (citric acid, malic acid, lactic acid, etc.) | 0.1 - 3.5 | 0.5 - 2.5 | 1.50 |
| Free black grape extract | 0.0006 - 3.0 | 0.06 - 1.5 | 0.60 |
| Black grape extract with encapsulation A | 0.0015 - 7.8 | 0.56 - 3.9 | 0.60 |
| Black grape extract with encapsulation B | 0.0013 - 6.77 | 0.135 - 3.38 | 0.60 |
| Flavourings | 0.05 - 0.5 | 0.1 - 0.3 | 0.20 |
| Colourants | 0.001 - 0.5 | 0.01 - 0.1 | 0.04 |
| | | | 100.00 |

In a particular embodiment of the present invention wherein the oral product is a hard candy, the sum of the different components is 100%. The adjustment to 100% of the total weight of the oral product is made for all combinations with sugar and/or polyols and syrups, maintaining the proportions established for them. Typically, combinations of sugar-polyols/glucose syrups/maltitol syrups are usually 50/50 and 60/40. Combinations of the three black grape extracts, combined in different%, or only the free extract can be used in this type of confectionery product.

It should be noted that since it is a hard candy which gradually dissolves in the mouth, free black grape extract alone would be sufficient to release the black grape extract progressively in the mouth during the dissolution time of the candy.

**Table 4. Table describing the amounts in which each of the components is comprised for a preferred embodiment, a more preferred embodiment and an even more preferred embodiment wherein the oral product is a chewy candy.**

| **STANDARD CHEWY CANDY WITH BLACK GRAPE EXTRACT** | | | |
|---|---|---|---|
| Product ingredients: With sugar/Sugar-free | A preferred embodiment (% by weight) | A more preferred embodiment (% by weight) | An even more preferred embodiment (% by weight) |
| Sugar/Polyols | ---- - ---- | ---- - ---- | 42.68 |
| Glucose syrup/Maltitol syrup | ---- - ---- | ---- - ---- | 42.68 |
| Hydrogenated vegetable fat | 2.00 - 12.00 | 4.00 - 8.00 | 6.00 |
| Gelling agents (gelatin, acacia gum, modified starches, maltodextrins, etc.) | 1.00 - 10.00 | 3.00 - 7.00 | 5.00 |
| Acids (citric acid, malic acid, lactic acid, etc.) | 0.10 - 3.50 | 0.50 - 2.50 | 1.50 |
| Free black grape extract | 0.0006 - 3.00 | 0.06 - 1.50 | 0.60 |
| Black grape extract with encapsulation A | 0.0015 - 7.80 | 0.56 - 3.90 | 0.60 |
| Black grape extract with encapsulation B | 0.0013 - 6.77 | 0.14 - 3.38 | 0.60 |
| Flavourings | 0.05 - 0.50 | 0.10 - 0.30 | 0.20 |
| Emulsifiers (lecithin, etc.) | 0.04 - 0.24 | 0.08 - 0.16 | 0.10 |
| Colourants | 0.00 - 0.50 | 0.01 - 0.10 | 0.04 |
| | | | 100.00 |

In this particular embodiment, the sum of the different components is 100%. The adjustment to 100% of the total weight of the oral product is made for all combinations with sugar and/or polyols and syrups, maintaining the proportions established for them. Combinations of the three black grape extracts, combined in different%, or only the free extract can be used in this type of confectionery product.

It should be noted that since it is a chewy candy which is held in the mouth for a certain period of time while chewing, free black grape extract alone would be sufficient to release the black grape extract progressively in the mouth during the dissolution time of the candy.

In a preferred embodiment of the present invention, the oral product is a gummy:

**Table 5. Table describing the amounts in which each of the components is comprised for a preferred embodiment, a more preferred embodiment and an even more preferred embodiment wherein the oral product is a gummy.**

| **STANDARD GUMMY WITH BLACK GRAPE EXTRACT** | | | |
|---|---|---|---|
| Product ingredients: With sugar/Sugar-free | A preferred embodiment (% by weight) | A more preferred embodiment (% by weight) | An even more preferred embodiment (% by weight) |
| Sugar/Polyols | ---- - ---- | ---- - ---- | 33.78 |
| Glucose syrup/Maltitol syrup | ---- - ---- | ---- - ---- | 50.67 |
| Gelling agents (gelatin, pectin, agar-agar, modified starches, carrageenan, etc.) | 3.00 - 14.00 | 6.00 - 10.00 | 8.00 |
| Wetting agent (sorbitol, glycerine, dextrose, etc.) | 0.50 - 8.00 | 2.00 - 6.00 | 4.00 |
| Acids (citric acid, malic acid, lactic acid, etc.) | 0.10 - 3.50 | 0.50 - 2.50 | 1.50 |
| Free black grape extract | 0.0006 - 3.00 | 0.06 - 1.50 | 0.60 |
| Black grape extract with encapsulation A | 0.0015 - 7.80 | 0.56 - 3.90 | 0.60 |
| Black grape extract with encapsulation B | 0.0013 - 6.77 | 0.14 - 3.38 | 0.60 |
| Flavourings | 0.05 - 0.50 | 0.10 - 0.30 | 0.20 |
| Colourants | 0.00 - 0.50 | 0.01 - 0.10 | 0.05 |
| | | | 100.00 |

In all cases, the sum of the different components is 100%. The adjustment to 100% of the total weight of the oral product is made for all combinations with sugar and/or polyols and syrups, maintaining the proportions established for them. Combinations of the three black grape extracts, combined in different%, or only the free extract can be used in this type of confectionery product. It should be noted that since it is a gummy, which is chewed for a short time, free black grape extract alone would be sufficient to release the black grape extract progressively in the mouth during the chewing and swallowing time of the product.

In a preferred embodiment of the present invention, the oral product is a chewing gum:

**Table 6. Table describing the amounts in which each of the components is comprised for a preferred embodiment, a more preferred embodiment and an even more preferred embodiment wherein the oral product is a chewing gum.**

| | A preferred embodiment (% by weight) | A more preferred embodiment (% by weight) | An even more preferred embodiment (% by weight) |
|---|---|---|---|
| Polyols/sugars | 50.00 - 75.00 | 59.0 - 69.00 | 64.32 |
| Gum base | 15.00 - 40.00 | 20.00 - 28.00 | 24.50 |
| Flavourings | 1.00 - 8.50 | 2.00 - 6.50 | 4.43 |
| Stabilisers | 0.10 - 6.50 | 0.50 - 2.50 | 1.25 |
| Acids | 0.50 - 8.00 | 1.00 - 5.00 | 2.66 |
| Free black grape extract | 0.0006 - 3.0 | 0.06 - 1.50 | 0.70 |
| Black grape extract encapsulation A | 0.0015 - 7.8 | 0.56 - 3.90 | 0.70 |
| Black grape extract encapsulation B | 0.0013 - 6.77 | 0.135 - 3.38 | 0.70 |
| Sweeteners | 0.05 - 0.60 | 0.20 - 0.50 | 0.46 |
| Glazing agent | 0.10 - 0.25 | 0.15 - 0.20 | 0.17 |
| Emulsifiers | 0.05 - 0.50 | 0.10 - 0.30 | 0.11 |
| Total by weight | | | 100.00 |

In particular embodiments of the present invention, the oral product comprises at least one additive selected from the group consisting of milk, cream, fruit juices, purées and liquorice extract, or a combination thereof.

In other particular embodiments, the oral product comprises glazing agents, waxes, sugars or acids on its surface, in order to give the surface of the product a gloss and prevent different parts of the product from sticking together.

In preferred embodiments, the oral product is a sugar-free product and comprises at least one sweetener, which is referred to in the state of the art as the "main sweetener in the formula" and is selected from the group consisting of sorbitol, maltitol, xylitol, isomaltose, mannitol, lactitol, erythritol, maltitol syrup and a combination thereof. In preferred embodiments, the sweetener is in an amount comprised between 40 - 85% by weight with respect to the total weight. In a more preferred embodiment, the sweetener is in an amount comprised between 50 - 75% by weight with respect to the total weight. In more preferred embodiments, the sweetener is in an amount comprised between 54 - 65% by weight with respect to the total weight.

In other preferred embodiments, the oral product is a sugar-free product, may comprise at least one high-intensity sweetener selected from the group consisting of aspartame, acesulfame K, sucralose, neohesperidin dihydrochalcone (NHDC), thaumatin, neotame, advantame, sodium and calcium saccharin, steviol glycosides, aspartame salt, acesulfame and a combination thereof. In preferred embodiments, the high-intensity sweetener is in an amount comprised between 0.005 - 0.5% by weight with respect to the total weight. In more preferred embodiments, the high-intensity sweetener is in an amount comprised between 0.01 - 0.1%, being even more preferably between 0.05-0.09%.

In preferred embodiments, the oral product is a product with sugar and comprises at least one sweetener selected from the group consisting of sucrose, dextrose, maltose, lactose, glucose syrup and a combination thereof. In preferred embodiments, the sweetener is in an amount comprised between 40 - 75% by weight with respect to the total weight. In a more preferred embodiment, the sweetener is in an amount comprised between 50 - 75%. In more preferred embodiments, the sweetener is in an amount comprised between 54 - 65% by weight with respect to the total weight.

In particular embodiments of the present invention, the oral product comprises gum base, which, in the context of the present invention, is a hydrogenated microcrystalline wax composed mostly of branched-chain hydrocarbons, preferably, a high iso-paraffin content, which give it a high degree of plasticity. In preferred embodiments, the amount of gum base comprised in the oral product is comprised between 15% and 40%. In more preferred embodiments, the amount of gum base comprised in the oral product is comprised between 20% and 32%. In even more preferred embodiments, the amount of gum base comprised in the oral product is 20%, 21%, 22%, 23%, 24%, 25%, 26%, 27%, 28%, 29%, 30%, 31% or 32%.

In particular embodiments of the present invention, the oral product comprises other ingredients, also referred to herein as additives and are selected from the group consisting of flavourings, acids, acacia gum, water, glazing agent, modified starch, emulsifier, gelatin or a combination thereof. In preferred embodiments, the amount of additives comprised in the oral product is comprised between 8% and 12%. In more preferred embodiments, the amount of additives comprised in the oral product is 8%, 8.5%, 9%, 9.5%, 10%, 10.5%, 11%, 11.5% or 12%.

In preferred embodiments of the present invention, when the oral product is a candy, the grape extract is in free form so the extract is released as soon as the candy is sucked. In preferred embodiments of the present invention, when the oral product is a candy, the grape extract in a free state is released for a time comprised from 5 to 15 minutes, which is the mean time it takes for a candy weighing 4 to 5 grams to dissolve in the mouth. In more preferred embodiments, the free grape extract is released for a time comprised from 5 to 10 minutes, being even more preferably from 7 to 9 minutes.

In a preferred embodiment of the invention, the oral product is a chewing gum, wherein the chewing gum further comprises a gum base and, optionally, one or more excipients selected from plasticisers, elastomers, softeners, fillers and bulking agents, or wherein the oral product is a lozenge, wherein the lozenge further comprises one or more excipients selected from sweetening and flavouring agents.

In particular embodiments, when the oral product is a candy or a lollipop, the grape extract is in a free state.

In particular embodiments, when the oral product is a chewing gum, the grape extract is free and encapsulated in type A and type B.

In particular embodiments of the present invention, the oral product is a dragée-type chewing gum and comprises one or more excipients selected from the group consisting of polyols, a gum base, flavourings, coating agents and stabilisers, acids, encapsulated acids, high-intensity sweeteners, glazing agent, emulsifiers and a combination thereof.

In an even more preferred embodiment of the present invention, the oral product is a chewing gum with the following composition:

**Table 7. Composition of a particular embodiment of dragée-type chewing gum according to the present invention.**

| **ANTIVIRUS DRAGÉE (1.4 g)** | |
|---|---|
| **AOM20220922-PA1** | **BERRIES** |
| Description | % |
| Isomaltose | 28.42 |
| Sorbitol powder | 24.44 |
| Gum base | 24.30 |
| Mannitol | 7.00 |
| Maltitol syrup | 4.80 |
| Berry flav. | 4.31 |
| Black grape extract | 1.28 |
| Acacia gum | 1.67 |
| Gelatin | 0.08 |
| Maltodextrin | 0.19 |
| Modified starch E-1450 | 0.13 |
| Anhydrous citric acid | 1.32 |
| Malic acid | 1.34 |
| Aspartame | 0.20 |
| Glazing agent | 0.16 |
| Sucralose | 0.12 |
| Acesulfame K | 0.13 |
| Lecithin | 0.11 |
| TOTAL | 100.00 |

**Table 8. Composition of a particular embodiment of a one-piece chewing gum according to the present invention.**

| **ANTIVIRUS CHEWING GUM PIECE FORMAT (4.0 g)** | |
|---|---|
| **AOM20220509-PA2** | **STRAWBERRY** |
| Description | % |
| Sorbitol | 41.33 |
| Gum base | 27.00 |
| Maltitol syrup | 11.00 |
| Mannitol | 10.00 |
| Glycerine | 3.00 |
| Liquid fruit flavouring | 1.45 |
| Powder fruit flavouring | 1.00 |
| Citric acid | 0.85 |
| Encapsulated citric acid | 0.72 |
| Free black grape extract | 0.60 |
| Black grape extract | |
| encapsulation A | 0.60 |
| Black grape extract | |
| encapsulation B | 0.60 |
| Malic acid powder | 0.55 |
| Encapsulated malic acid | 0.48 |
| Aspartame E-951 | 0.35 |
| Sucralose E-955 | 0.22 |
| Acesulfame K E-950 | 0.15 |
| Sunflower lecithin | 0.10 |
| | 100.00 |

The weights described are as a percentage with respect to the total weight.

In particular embodiments of the present invention, the oral product of the present invention is a chewing gum and comprises at least one polyol. In preferred embodiments of the present invention, the at least one polyol is selected from the group consisting of sorbitol, maltitol, mannitol, xylitol, isomaltose, lactitol, erythritol, maltitol syrup or a combination thereof. In even more preferred embodiments, the at least one polyol is selected from the group consisting of sorbitol, maltitol, maltitol syrup or a combination thereof. In the scope of the present invention, polyols are hydrogenated sugars and have the technical advantage that they do not ferment in the oral cavity. To that end, these products maintain a pH in the mouth above the critical value for tooth enamel wear. As long as the enamel protects the tooth, the bacteria that cause tooth decay cannot affect the teeth, as they are unable to act. Likewise, polyols also have the advantage over sugars in providing fewer calories to the consumer. In other words, polyols provide about 2.4 cal/g, while sugar, which is sucrose, provides 4 cal/g, compared to 9 cal/g for fat and 7 cal/g for alcohol.

In particular embodiments of the present invention, the oral product of the present invention comprises at least one flavouring selected from the group consisting of fruit flavourings, mints, chamomile, vanilla, violet, cinnamon or a combination thereof.

In preferred embodiments, the fruit flavouring is selected from the group consisting of berries, citrus, strawberry, apple, banana, pear, cherry, pineapple, mango, passion fruit, pomegranate, lychee, custard apple, currant, raspberry, blackberry, watermelon, melon, grape, peach or a combination thereof. In more preferred embodiments, the citrus flavouring is selected from the group consisting of orange, lemon, grapefruit, mandarin or a combination thereof.

In more preferred embodiments, the mint flavouring is selected from the group consisting of peppermint, spearmint, chlorophyll, menthol and a combination thereof.

In particular embodiments of the present invention, the oral product comprises at least one coating agent selected from the group consisting of carnauba wax, beeswax, candelilla wax and shellac, or a combination thereof.

In particular embodiments of the present invention, the oral product comprises at least one acid selected from the group consisting of citric acid, malic acid, tartaric acid, acetic acid, lactic acid, ascorbic acid, phosphoric acid and a combination thereof. In more preferred embodiments, the oral product comprises at least one acid selected from the group consisting of citric acid, malic acid and a combination thereof.

In preferred embodiments of the present invention, the oral product is a chewing gum and comprises at least one encapsulated acid. In preferred embodiments of the present invention, the at least one encapsulated acid is, in preferred embodiments, encapsulated by fats with a different melting point. In more preferred embodiments, these fats are stearins with a high or very high melting point, preferably between 60°C and 70°C, or more. In other preferred embodiments, the acid is coated with other coating materials selected from the group consisting of acacia gum, modified starches or a combination thereof. The technical effect of such materials is that they allow the slow release of the acid while chewing of the gum. This makes the sour note in the chewing gum last longer, giving a longer duration to that sour effect. In sour flavours, the sour note enhances the flavour of the fruit in the chewing gum.

In particular embodiments of the present invention, the oral product comprises at least one high-intensity sweetener selected from the group consisting of aspartame, acesulfame K, sucralose, neohesperidin dihydrochalcone (NHDC), thaumatin, neotame, advantame, sodium and calcium saccharin, steviol glycosides, aspartame salt, acesulfame and a combination thereof.

In particular embodiments of the present invention, the oral product comprises at least one glazing agent selected from the group consisting of carnauba wax, white and yellow beeswax, candelilla wax, microcrystalline wax, shellac or a combination thereof.

In particular embodiments of the present invention, the oral product comprises at least one emulsifier selected from the group consisting of soya lecithin, sunflower lecithin, mono- and diglycerides of fatty acids and a combination thereof.

In particular embodiments of the present invention, the oral product comprises at least one wetting agent selected from the group consisting of glycerol or glycerine, sorbitol and a combination thereof. In preferred embodiments, the wetting agent is glycerine.

In particular embodiments of the present invention, the oral product comprises at least one modified starch selected from the group consisting of starch sodium octenyl succinate, acetylated starch, acetylated distarch adipate, hydroxypropyl starch, hydroxypropyl distarch phosphate and a combination thereof.

In particular embodiments of the present invention, the oral product comprises at least one non-stick agent selected from the group consisting of talc, sugar, mannitol, starches, isomaltose and a combination thereof. More preferably, the non-stick agent is talc, which is used to prevent the chewing gum from sticking to surfaces in the lamination, cutting and packaging process.

In particular embodiments of the present invention, the oral product comprises at least one gelling agent selected from the group consisting of pork gelatin, cow gelatin, fish gelatin, agar-agar, pectin and a combination thereof.

In a second aspect, the invention relates to the use of an oral product comprising black grape extract as an antiviral agent.

In a preferred embodiment, the invention relates to the use of the oral product for reducing and/or eliminating the viral load in the oral cavity, in the respiratory tract, preferably in the upper airways, and in the exhaled air.

In the context of the present invention, viral load is understood to mean the quantification of virus infection calculated by estimating the number of viral particles in body fluids.

In a preferred embodiment of the present invention, said viral load is a viral load of a coronavirus, preferably, cold coronavirus 229E.

In another preferred embodiment, the invention relates to the use of the oral product wherein said oral product is held inside in the oral cavity for a time comprised from 5 to 60 minutes.

Likewise, another object of the invention is the oral product according to the first aspect of the invention for use in the prevention of coronavirus infection.

In a preferred embodiment of the present invention, the inventors have found that the use of this oral product has surprisingly reduced the viral load of cold coronavirus 229E, obtaining an extremely positive result of inhibition and/or destruction of the said virus with the oral product object of the invention, which comprises very small amounts of black grape extract.

In a preferred embodiment of the invention, the oral product is held in the oral cavity to be chewed or sucked or to dissolve in the oral cavity. In more preferred embodiments of the present invention, the oral product is held inside the oral cavity for a time comprised from 1 to 60 minutes. In preferred embodiments, the time is comprised from 5 to 30 minutes, while in another more preferred embodiment, the time is comprised from 10 to 30 minutes, more preferably, from 10 to 15 minutes.

In particular embodiments, the oral product is a chewing gum comprising black grape extract in three forms, a free or non-encapsulated form and in an encapsulated form. The free form is released very quickly as the chewing gum is hydrated by saliva. To that end, grape extract in a free state starts to be detectable from the first minute of chewing, which in more particular embodiments is a time of 0.5 to 1 minute and up to a maximum time of 2 to 5 minutes. After this time, the grape extract that is released is the grape extract that is encapsulated. To that end, in particular embodiments wherein the chewing gum comprises grape extract in the two types of encapsulation, after release of the extract that is in free form, the grape extract that is encapsulated in the type A capsule, which contains maltodextrin, is released. Next, the grape extract that is encapsulated in the type B capsule, which contains gelatin, is released. In preferred embodiments, the release lasts for more than 15 minutes. After the first 15 to 30 minutes of chewing, it is considered that more than a sufficient amount of the black grape extract to eliminate the virus has already been released.

Based on the above, when the oral product is a chewing gum, the release time of the grape extract will be comprised from 0 to more than 30 minutes, even 45 minutes or 60 minutes.

In other preferred embodiments, when the oral product is a hard candy weighing about 10 grams, the release time of the grape extract will be comprised from 0 to 15 minutes, preferably in a time comprised from 5 to 15 minutes, than which is the time that must elapse for the candy to be completely dissolved.

In other preferred embodiments, when the oral product is a chewy candy or a gummy, the release time of the grape extract will be comprised from 0 to about 2 to 5 minutes. In preferred embodiments, the chewy candy dissolves faster than a hard candy. In other embodiments, the gummy dissolves even faster than the candies, since, by chewing it, it is broken down into small pieces, which makes it dissolve even faster than the candies.

After numerous studies, the inventors have found that this oral product comprising grape extract is surprisingly effective in inhibiting and even eliminating the viral load in the oral cavity in very small amounts. This is thanks to the design of the oral product described in this document, wherein a very small amount of grape extract is strategically released while chewing, in the preferred case of chewing gum or while consuming a gel, candy or lozenge.

In the context of the present invention, the gum base is a substance designed to be chewed without being swallowed; other components are usually added to same in order to market it as chewing gum with different flavours and colours, giving it a pleasant appearance for the consumer.

The term "effective amount", as it is used herein, means an amount of an active agent high enough to achieve the desired benefit, but small enough to avoid serious side effects within the scope of good medical judgement.

After numerous tests, researchers have succeeded in developing an oral antiviral product that exhibits an inhibitory effect on the infectivity of coronavirus-type viruses, and in particular, the HuCov-229E coronavirus, which is one of the typical cold coronaviruses.

Throughout the description and claims, the word "comprises" and its variations are not intended to exclude other technical features, additives, components or technical steps. Additional objects, advantages and features of the invention will be apparent to those skilled in the art upon examination of the description or may be learnt by means of practice of the invention. Furthermore, the present invention encompasses all the possible combinations of particular and preferred embodiments described herein.

### EXAMPLES

The examples indicated below are for illustrative purposes and are not intended to limit the scope of the invention in any way.

### Example 1. Determination of the inhibitory potential of a grape extract on human coronavirus (229E) infectivity

### Objective of the study

In this study, the ability of the product object of the invention to inhibit the infectivity of human coronavirus 229E was determined. To that end, different concentrations of grape extract were incubated together with a defined concentration of the virus of interest for 30 minutes. Exposure of host cells to the virus causes the virus to replicate within the cells, which produces a cytopathic effect on the cells that is quantified by means of a cell viability assay after 5 days of exposure. Therefore, it was assessed whether the grape extract had an inhibitory effect on the infectivity of the virus of interest; cells treated with the test substance-virus mixture will not show any reduction in cell viability.

The black grape extract tested is characterised in that it comprises:
- 100% total catechin-equivalent polyphenols in a 280nm OD analysis;
- 70% gallic acid equivalents in a Folin-Ciacalteu analysis; and
- 2% anthocyanin (cyanidin-3-O-glucoside) equivalents in an HPLC-DAD analysis.

### Cell cultures and viruses

The MRC-5 cell line (lung-derived fibroblasts) from the American Type Culture Collection (ATCC) (CCL-171) was used. Cells were kept at 37°C and 5% CO2 in Eagle minimum essential medium (EMEM) supplemented with 10% foetal bovine serum (FBS) and antibiotics, trypsinising every 3-4 days, before reaching 90% confluence. Coronavirus 229E was obtained from the ATCC (VR-740) and amplified in MRC-5 cells, according to the ATCC protocol.

### 229E infectivity inhibition assays

For 229E infectivity inhibition assays, MRC-5 cells were seeded in 96-well plates at a density of 5000 cells/well, 48 hours before treatments.

Two experiments were carried out with different test substance concentration ranges:

### Experiment 1

A stock solution of the test substance was prepared in dimethyl sulfoxide (DMSO) at 100 mg/ml. From the stock solution, a 2 mg/ml solution in phosphate buffer saline (PBS) was prepared, along with 1/2 serial dilutions down to 0.015625 mg/ml. Next, 0.25 ml of each dilution were mixed with 0.25 ml of a 229E inoculum diluted in 2% FBS EMEM medium, with the inoculum at an infectious concentration of 1000 TCID50/ml and the maximum concentration of the extract at 1 mg/ml (concentration range analysed in the medium: 7.81-1000 µg/ml). The test substance-inoculum mixture was incubated for 30 minutes at room temperature. The medium was aspirated from the wells and 0.1 ml of each test substance-inoculum mixture was added in quadruplicate. It was incubated for 4.5h at 35°C and 5% CO2 to allow adsorption of the virus by the MRC-5 cells. Next, the medium was aspirated from the cells, 2% FBS EMEM medium was added and the cells were incubated for 5 days at 35°C and 5% CO2.

In all treatments, the final concentration of DMSO in the medium during the 4.5h of treatment was 1%.

### Experiment 2

Due to the fact that the minimum amount of black grape extract effective against the virus could not be defined in Experiment 1, another experiment was performed with amounts below 8 µg/ml to be able to define the minimum amount of grape extract needed to inactivate the virus.

To that end, a stock solution of the test substance was prepared in dimethyl sulfoxide (DMSO) at 50 mg/ml. From the stock solution, a 200 µg/ml solution in PBS was prepared, along with 1/2 serial dilutions down to 1.5625 µg/ml. Next, 0.25 ml of each dilution were mixed with 0.25 ml of a 229E inoculum diluted in 2% FBS EMEM medium, with the inoculum at an infectious concentration of 1000 TCID50/ml and the maximum concentration of the extract at 1 mg/ml (concentration range analysed in the medium: 0.78125 µg/ml - 100 µg/ml). The test substance-inoculum mixture was incubated for 30 minutes at room temperature. The medium was aspirated from the wells and 0.1 ml of each test substance-inoculum mixture was added in quadruplicate. It was incubated for 4.5h at 35°C and 5% CO2 to allow adsorption of the virus by the MRC-5 cells. Next, the medium was aspirated from the cells, 2% FBS EMEM medium was added and the cells were incubated for 5 days at 35°C and 5% CO2.

In all treatments, the final concentration of DMSO in the medium during the 4.5h of treatment was 0.2%.

Toxicity controls were performed for all concentrations of the test substance and chloroquine by mixing 0.25 ml of each concentration with 0.25 ml of virus-free 2% FBS EMEM medium to determine the toxicity associated with the test substance.

As a positive control for the experiments, chloroquine (Sigma C6628) was used, performing the same procedure as described for the test substance and with a final concentration range in the medium of 0.0625 - 256 µg/ml.

In both experiments, 5 days post-infection, differences in cell viability associated with the cytopathic effect of the virus or toxicity of the treatments were analysed by means of the MTT assay. The MTT assay is a colorimetric assay that measures the activity of enzymes that transform the MTT (methylthiazolyldiphenyl-tetrazolium bromide) molecule to formazan, purple in colour. Briefly, the medium was aspirated from the wells and 0.1 ml of 1 mg/ml MTT solution in EMEM was added to each well, and incubated at 37°C for 2 hours. Next, the MTT solution was aspirated, formazan crystals were dissolved in 0.1 ml of DMSO and absorbance was read at 570 nm (reference 660 nm). The mean absorbance values of the uninfected controls was considered to be 100% cell viability.

### Calculation of results.

In order to evaluate the results, the following variables were calculated:
- CC50: Half maximal cytotoxic concentration, which is the concentration of the compound of interest causing 50% toxicity. Low CC50 values indicate higher toxicity.
- EC50: Half maximal effective concentration, which is the concentration of the compound of interest that inhibits the toxicity caused by the viral infection by 50%. Low EC50 values indicate higher inhibition.
- SI: Selectivity index, which is calculated by dividing the CC50 by the EC50. The higher the SI, the better the toxicity/effect ratio of the compound.

CC₅₀ and EC₅₀ values were obtained by means of non-linear regressions with GraphPad Prism 9.0.1 software.

### Analysis of the results

In the first experiment, in which a grape extract dose range of 7.81-1000 µg/ml was tested, an inhibitory effect on HuCov-229E infectivity was observed at all tested doses of the extract which did not show a decrease in cell viability in their cytotoxicity control (without 229E virus), as no decrease in cell viability associated with viral infection was observed at any dose. A decrease in cell viability was observed both in cells treated with extract alone and in cells treated with the virus and the extract starting from 500 µg/ml, with near-total toxicity at a concentration of 1000 µg/ml. This allowed the CC50 (theoretical concentration of the extract causing 50% toxicity) of the extract to be established at 529.3 µg/ml (Figure 1).

Like in the first experiment, no toxicity associated with viral infection was observed at low doses of the extract, and in order to be able to establish the minimum concentration at which inhibition and the EC50 (theoretical effective concentration that inhibits the toxicity associated with viral infection by 50%) of the extract occurred, a second experiment was performed with a lower grape extract dose range: 0.781-100 µg/ml. Said experiment confirmed that certain inhibition of the cytopathic effect of HuCov-229E virus occurs starting from 1.56 µg/ml of grape extract in the medium, with total inhibition starting from 6.25 µg/ml, and it allowed an EC50 of 2.634 µg/ml to be established (Figure 2).

As for the results of the antiviral positive control used in this study, that is, chloroquine, a clear antiviral effect starting from 16.5 µg/ml was observed, with an EC₅₀ of 5.96 µg/ml. In the dose range used (0.06-264 µg/ml), no significant toxicity was observed at any dose, so the CC₅₀ of chloroquine was higher than 264 µg/ml (Figure 3).

In summary, the EC₅₀ and CC₅₀ values shown in Table 9 were obtained for chloroquine and grape extract. The selectivity index (SI) of each compound, which is indicative of the ratio of toxic concentration to effective concentration, was calculated. Therefore, grape extract showed a good SI for the tested conditions, as the CC₅₀ was 200 times higher than the EC₅₀, which means that effective antiviral concentrations are far from toxic concentrations. Interestingly, it should be noted that grape extract showed antiviral effectiveness at lower doses than chloroquine, a well-known drug used as a treatment against malaria, which has shown *in vitro* antiviral activity against coronaviruses such as SARS-CoV, MERS-CoV, HCoV-OC43 and HuCov-229E_{,} and which was proposed as a treatment against SARS-COV-2 infection in the initial stages of the pandemic.

**Table 9. EC₅₀, CC₅₀ and SI for values for chloroquine and grape extract.**

| | **EC50 (µg/ml)** | **CC50 (µg/ml)** | **SI** |
|---|---|---|---|
| **Chloroquine** | 5.96 | >264 | >44.3 |
| **Grape extract** | 2,634 | 529.3 | 200.9 |

### Conclusions of the study

The results of the present study have demonstrated a clear antiviral effect of the grape extract tested, with a total inhibition of the cytopathic effect of HuCoV-229E infection starting from 6.25 µg/ml. The extract has shown antiviral effectiveness at lower doses than chloroquine, a drug with known antiviral properties.

It should be noted that the concentrations determined in the present study are effective for the *in vitro* conditions described, in which antiviral effectiveness was tested against an infective dose of 100 TCID50/reaction, and that higher concentrations of the virus may require a higher concentration of grape extract to have the same effectiveness.

The present study shows the concentrations from which grape extract shows inhibitory activity on HuCoV-229E infectivity. Once the minimum concentrations to obtain an antiviral effect have been determined, as possible future steps, it would be interesting to determine the percentage reduction in the infective load of one or two specific doses, such as 25 or 50 µg/ml, exposing the grape extract to a higher viral concentration -106 TCID50- and determining the percentage inhibition of infectivity. These additional studies would provide further insight into the antiviral effect of the grape extract, which could be used as a starting point to obtain further sound scientific evidence for the potential use of this natural ingredient as a strategy to help prevent viral infections or as adjunctive therapy when infection has already occurred.

### Example 2. Preparation of a linear regression table between absorbance and concentration (ppm) of black grape extract.

### Objective of the study

To find out the relationship between absorbance and concentration in ppm of grape extract. To that end, by means of a standard procedure, a table with grape extract samples is prepared, and subsequently the absorbance of the sample is measured to be able to prepare the linear regression line where the correlation between absorbance and concentration of black grape extract is observed and which is depicted in figure 4.

The results are also shown in the following table:

**Table 10. Table showing linear regression between absorbance and grape extract concentration (ppm).**

| | ppm of black grape extract | Absorbance |
|---|---|---|
| P0 | 0 ppm | 0 |
| P1 | 2 ppm | 0.158 |
| P2 | 4 ppm | 0.198 |
| P3 | 8 ppm | 0.277 |
| P4 | 16 ppm | 0.396 |

According to this graph, it is known that only 2 ppm of black grape extract leads to an absorbance result of 0.158. Furthermore, the increase in absorbance is correlated to the increase in ppm of black grape extract. A good correlation between ppm of black grape extract and absorbance can be observed in the graph.

Moreover, according to the virological study performed with coronavirus 229E, it has been demonstrated that 6.25 ppm of black grape extract completely inactivates the 229E coronavirus population.

Therefore, by extrapolating on the correlation graph between absorbance and ppm concentration of black grape extract, 6.25 ppm of black grape extract would correspond approximately to an absorbance of 0.263, which would correspond to the minimum amount of ppm of black grape extract needed to completely inactivate the 229E coronavirus population.

### Example 3. Analysis of the amount of black grape extract released into the oral cavity by chewing a piece of chewing gum.

### Objective of the study

To find out the amount of black grape extract released by the oral product, and in this case, a dragée-type chewing gum as described herein and in particular in Table 7 (AOM20220922-PA1- dragée-type chewing gum 1.4 g).

Seven replicate samples of each sample were analysed according to the European Pharmacopoeia in order to be able to subsequently perform the statistical study.

For this analysis, the dragée-type chewing gum described in Table 7 of the present document was analysed. A chewing test that simulates the chewing that takes place inside the oral cavity was performed. To that end, the chewing protocols and machinery as established in European Pharmacopoeia 7.4 Vol.04/2012:20925 were followed. Preferably, the machinery described in this protocol, Chapter 2.9.25 - Type B machinery, was used.

The conditions under which said test was carried out are as follows:
- Solvent used: Phosphate buffer pH=6
- Solvent volume: 20 ml
- Temperature of mastication medium: 37.0±0.5°C
- No. of chews: 60 chews/min
- Sampling time: 2, 5, 10, 18 and 30 minutes

The solvent used to carry out the study had the following composition:
- Na2HPO4.12H₂O: 28.6520 g
- C12H25NaO₄S: 1.0049 g

Table 11 below shows the total weight of each of the samples evaluated in the analytical study comprising 2 dragées and the solvent:

**Table 11. Descriptive table indicating the weight in grams of the samples evaluated for this analytical study and the weight of the solvent.**

| **ANTIVIRUS AOM20220922-PA1 CHEWING GUM RELEASE STUDY** | | | |
|---|---|---|---|
| **Chewing Time (min)** | **Sample identifier** | **Weight of 2 dragées** | **Solvent weight** |
| **2** | **1** | 2.71 | 19.95 |
| | **2** | 2.83 | 20.03 |
| | **3** | 2.89 | 20.08 |
| | **4** | 2.83 | 20.10 |
| | **5** | 2.93 | 20.01 |
| | **6** | 2.81 | 19.92 |
| | **7** | 2.75 | 19.97 |
| **5** | **1** | 2.79 | 19.95 |
| | **2** | 2.93 | 19.93 |
| | **3** | 2.83 | 20.05 |
| | **4** | 2.79 | 20.06 |
| | **5** | 2.84 | 19.90 |
| | **6** | 2.76 | 19.98 |
| | **7** | 2.81 | 20.05 |
| **10** | **1** | 2.86 | 19.90 |
| | **2** | 2.84 | 20.03 |
| | **3** | 2.91 | 20.08 |
| | **4** | 2.84 | 20.12 |
| | **5** | 2.88 | 19.98 |
| | **6** | 2.87 | 19.92 |
| | **7** | 2.86 | 19.89 |
| **18** | **1** | 2.86 | 20.09 |
| | **2** | 2.92 | 19.93 |
| | **3** | 2.83 | 19.92 |
| | **4** | 2.85 | 20.08 |
| | **5** | 2.88 | 20.00 |
| | **6** | 2.76 | 19.95 |
| | **7** | 2.84 | 20.12 |
| **30** | **1** | 2.87 | 19.90 |
| | **2** | 2.90 | 19.92 |
| | **3** | 2.87 | 19.97 |
| | **4** | 2.86 | 20.09 |
| | **5** | 2.90 | 20.10 |
| | **6** | 2.78 | 20.08 |
| | **7** | 2.97 | 20.03 |

### Results

It can be observed in Figure 5 how after 2 minutes of chewing time, the absorbance already has a value of 1.78. In this regard, it should be noted that an absorbance of 0.263, which is equivalent to 6.25 ppm of black grape extract, is a sufficient amount to inactivate the virus. To that end, it is concluded that after 2 minutes, or most likely sooner, the amount of black grape extract released from the chewing gum while chewing is already far greater than what is needed to eliminate the entire population of 229E coronavirus in the mouth and retronasal cavity, i.e. about 42.3 ppm of black grape extract, when the entire coronavirus population is already eliminated with 6.25 ppm.

In the chewing time after the initial 2 minutes, and according to the test time of 30 minutes of maximum chewing, during the entire chewing time, the amount of black grape extract in the mouth is maintained at amounts far greater than those necessary to completely inactivate the coronavirus.

Having regard to the results of this test, which are expressed in the graph, it can be concluded that even if the chewing gum is chewed for more than 30 minutes, a sufficiently high amount of black grape extract would still be maintained to fully protect against the coronavirus under study.

## Claims

1. An oral antiviral product, **characterised in that** it comprises black grape extract.

2. The oral antiviral product according to claim 1, wherein the grape extract is encapsulated in a capsule selected from the group consisting of capsule with maltodextrin, a capsule with gelatin and a combination of the two.

3. The oral antiviral product according to claim 1, wherein the oral product comprises an amount of 0.0006 - 9% of black grape extract, by weight with respect to the total weight.

4. The oral antiviral product according to claim 1 or 2, wherein the maltodextrin capsule comprises an amount comprised from 9 to 42% maltodextrin, as a percentage by weight with respect to the total weight.

5. The oral antiviral product according to claim 1 or 2, wherein the gelatin capsule comprises an amount comprised from 4 to 20% gelatin, as a percentage by weight with respect to the total weight.

6. The oral antiviral product according to any one of claims 1 to 5, wherein the oral product is selected from the group consisting of a chewing gum, a lozenge, a gummy, a gel and a candy.

7. The oral antiviral product according to any of claims 1 to 6, wherein the oral product is a chewing gum and comprises other excipients selected from the group consisting of polyols, a gum base, flavourings, coating agents and stabilisers, acids, wastewater, encapsulated acids, high-intensity sweeteners, glazing agent, emulsifiers and a combination thereof.

8. Use of an oral product comprising black grape extract as an antiviral agent.

9. The use according to claim 8, for reducing and/or eliminating the viral load in the oral cavity, in the respiratory tract, preferably in the upper airways, and in the exhaled air.

10. The use according to claim 9, wherein said viral load is a viral load of a coronavirus, preferably, cold coronavirus 229E.

11. The use according to any one of claims 8 to 10, wherein the oral product is held inside the oral cavity for a time comprised from 5 to 60 minutes.

12. The oral antiviral product according to any one of claims 1 to 7, for use in the prevention of coronavirus infection.
